# EUROPÄISCHE PATENTSCHRIFT

(11) **EP 0 692 228 B1**
(45) Veröffentlichungstag und Bekanntmachung des Hinweises auf die Patenterteilung: **22.12.1999**
(21) Anmeldenummer: 95110275.5
(22) Anmeldetag: 01.07.1995
(51) Int. Cl.: A61F 2/46, A61F 2/30, A61F 2/36

(54) **Femurprothese**
Femoral prosthesis
Prothèse fémorale

(30) Priorität: 14.07.1994 DE 4424883
(43) Veröffentlichungstag der Anmeldung: 17.01.1996
(73) Patentinhaber: MERCK PATENT GmbH, 64293 Darmstadt (DE)
(72) Erfinder: Nies, Berthold, Dr., D-64407 Frankisch-Crumbach (DE)

(56) Entgegenhaltungen:
- EP-A- 0 423 064
- WO-A-91/08720
- WO-A-92/19200
- DE-A- 4 228 317
- FR-A- 2 629 337
- US-A- 4 888 022
- US-A- 4 888 024

## Beschreibung

Die Erfindung betrifft eine Endoprothese für den Hüftgelenkersatz, genau genommen den zugehörigen Prothesenschaft für die Implantation in das Femur, im weiteren kurz als "Femurprothese" bezeichnet.

Eine Femurprothese nach dem Oberbegriff von Anspruch 1 ist allgemein im Stand der Technik bekannt.

Für den Einsatz von Femurprothesen stehen bekanntlich zwei Implantationstechniken zur Verfügung. Zum einen kann die Implantation von Femurprothesen in den entsprechend vorbereiteten proximalen Bereich des Femur mittels Zementationstechnik erfolgen. Hierbei erfolgt die Verankerung des Prothesenschaftes im Knochenbett formschlüssig mit Hilfe von Knochenzement. Zum anderen kann die Implantation entsprechend gestalteter Femurprothesen durch zementlose Technik erfolgen. Hierbei wird für eine kraftschlüssige Verankerung ("Pressfit") des Prothesenschaftes mit dem Knochenbett gesorgt.

Die vorliegende Erfindung richtet sich auf eine Femurprothese, die für eine Implantation mittels Zementationstechnik vorgesehen ist und hier bezüglich Operationstechnik, Implantationsergebnis und Standzeit der Prothese erhebliche Verbesserungen mit sich bringt.

Trotz inzwischen hohem Entwicklungsstand bezüglich Optimierung von Prothesendesign, Zusammensetzung und Verarbeitungscharakteristik von Knochenzementsystemen, Operations- und Zementiertechnik ist das Implantationsergebnis immer noch von vielen situationsbedingten Faktoren beeinflußt und darüber hinaus in hohem Maße vom Können des Operateurs abhängig. Wesentliche Problempunkte hierbei sind die anatomiegerechte Zentrierung und Fixierung der Prothese und die situationsentsprechende Wahl der Zementcharakteristik.

Bei der Anwendung handelsüblicher Knochenzemente hoher Viskosität ist das Einsetzen, Zentrieren und Fixieren der Prothese in das vorbereitete und mit einem Zementbett versehene Knochenlager einfacher und sicherer durchführbar, da Lageveränderungen der Prothese bis zum Ende des Aushärtungsvorganges kaum mehr eintreten können. Allerdings ist das homogene und porenfreie Anmischen hochviskoser Knochenzemente schwierig. Die Applikation, etwa mit einer Knochenzementpresse oder Spritze, erfordert einen hohen Kraftaufwand. Außerdem ist es kaum möglich aufgrund der geringen Fließfähigkeit, einen allseits geschlossenen Zementmantel zwischen Knochenlager und Prothesenoberfläche zu erzeugen. Gerade dies aber ist für einen festen und dauerhaften Verbund und damit für eine lange Standzeit der Prothese unbedingt erforderlich.

Niedrigviskose, leichtflüssige Knochenzemente, wie sie ebenfalls handelsüblich sind, lassen sich in dieser Hinsicht erheblich leichter und sicherer verarbeiten. Allerdings gestaltet sich die Positionierung und Fixierung der Prothese schwierig, so daß in aller Regel zusätzliche Maßnahmen zur Stabilisierung des Prothesensitzes getroffen werden müssen.

Weiterentwicklungen bei der Zementierung mit niedrigviskosem Zement gehen dahin, daß man zuerst die Prothese in das Knochenlager einsetzt und dort mit Hilfe von Zentriervorrichtungen korrekt positioniert und danach erst den freibleibenden Raum im Knochenbett mit Zement hinterfüllt. Die Applikation des Knochenzements kann hierbei mittels Zementspritze und/oder Einsaugen mit Hilfe von Vakuum erfolgen. In einer weiteren Ausgestaltung dieser Zementiertechnik erfolgt die Zementierung nach dem Einsetzen und Fixieren der Prothese durch transprothetische Applikation. Hierfür geeignete Femurprothesen weisen eine von proximal bis distal reichende Längsbohrung des Schaftes auf, durch die der Knochenzement gespritzt wird, um dann das Knochenlager von distal bis proximal fortschreitend zu verfüllen. Beispielhaft für diese Technik sei auf EP 0 434 604 A1 verwiesen. Aber auch diese Technik birgt noch Nachteile, die sich auf den Operationserfolg und die Standzeit der Prothese auswirken können. So kann beispielsweise nicht ausgeschlossen werden, daß die Prothesenoberfläche mit Blut und Sekret und/oder Spülflüssigkeit kontaminiert wird oder daß ein Einbluten in den applizierten, im Vorgang des Aushärtens befindlichen Zementmantel erfolgt. Hierdurch wird der Verbund Prothese/Zementmantel/Knochenbett erheblich geschwächt, so daß es zu vorzeitiger Lockerung und Ausbruch des Implantates kommen kann.

Aufgabe der Erfindung ist es eine für die transprothetische Applikation geeignete Femurprothese so auszubilden, daß eine Kontamination von Prothesenoberfläche und appliziertem Zementmantel mit Blut, Wundsekret und Spülflüssigkeit nicht erfolgen kann, und daß sich eine besonders innige Verbindung zwischen der Knochensubstanz und dem Zement ausbildet, so daß der Verbund Knochenlager/Zement/Prothese weiter verstärkt wird.

Diese Aufgabe wird erfindungsgemäß durch die Femurprothese nach Anspruch 1 gelöst.

Gegenstand der Erfindung ist demnach eine Femurprothese für die Implantation mittels Zementierung, wobei deren Schaft eine von proximal bis distal reichende Längsbohrung zur transprothetischen Applikation des Knochenzements aufweist und der Prothesenschaft von einer dehnbaren Hülle aus einem biokompatiblen Polymermaterial in der Weise umgeben ist, daß sie bei Applikation des Zements von distal bis proximal fortschreitend hinterfüllt und bei vollständiger Befüllung des Raumes zwischen Schaff und Knochenlager bündig gegen das Knochenlager gepreßt wird. Die dehnbare Hülle weist Poren auf, die sich bei der Hinterfüllung der Hülle mit Knochenzement aufweiten, so daß der Zement mit dem Knochenlager in Kontakt tritt.

Als Materialien für die den Prothesenschaft umgebende dehnbare Hülle kommen eine Reihe von hochmolekularen Materialien natürlicher Herkunft in Betracht, die bioresorbierbar sind, also vom Organismus abgebaut und gegebenenfalls zu körpereigenen Stoffen umgewandelt werden. Dies trifft zu für Materialien auf Basis von Polylaktiden und Polyglykoliden sowie auf Biopolymere auf Basis von Polypeptiden oder Polysacchariden wie etwa Kollagen, Chitin und Chitosan.

Nachfolgend wird die Erfindung anhand der Zeichnung beispielhaft näher erläutert.

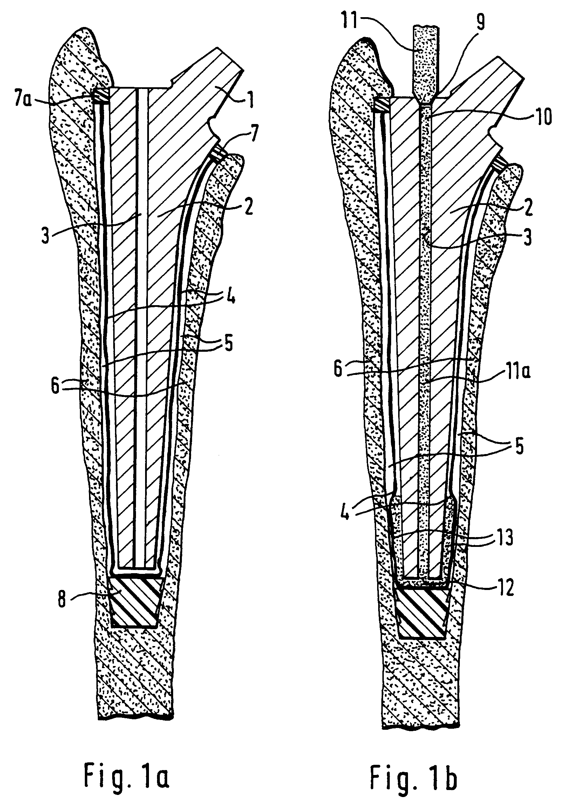

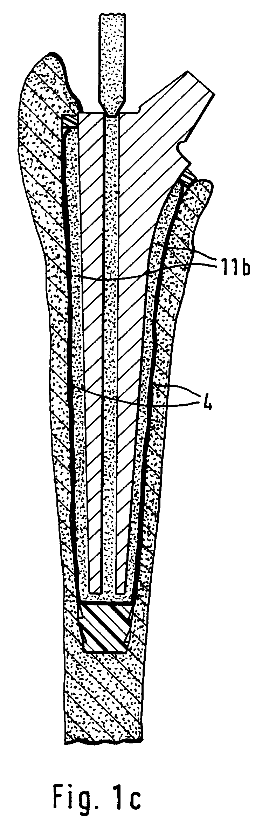

Es zeigt Fig. 1 schematisch in drei aufeinanderfolgenden Phasen (a), (b) und (c) den Zementierungsvorgang der erfindungsgemäßen Femurprothese.

In Phase (a) ist die Femurprothese 1 bereits in das durch Entfernung des Gelenkkopfes und Ausräumen von Spongiosa entsprechend vorbereiteten Fermur eingesetzt. Der Schaft 2 der Prothese weist eine von proximal bis distal reichende Längsbohrung 3 auf. Der Prothesenschaft 2 ist von einer im wesentlichen eng anliegenden Hülle 4 aus einem biokompatiblen Polymermaterial umgeben. Der verbleibende Raum 5 zwischen Schaft 2 und Knochenlager 6 ist zur Verfüllung mit Knochenzement vorgesehen. Im proximalen Bereich sitzen Abstandshalter 7, 7a zur Positionierung und Fixierung der Prothese. Unterhalb des distalen Endes des Prothesenschaftes verhindert ein Markraumsperrer 8 das Eindringen des zu applizierenden Knochenzementes in den Markraum.

In Phase (b) ist die Austrittsöffnung 9 einer Knochenzementspritze (nicht gezeigt) an der proximalen Öffnung 10 der Längsbohrung 3 des Prothesenschaftes 2 angesetzt. Knochenzement 11, 11a hat die Längsbohrung 3 erfüllt und tritt aus der distalen Öffnung 12 aus und beginnt von distal bis proximal fortschreitend 13 den Raum 5 zwischen Schaft 3 und Knochenlager 6 zur erfüllen, wobei die elastische Hülle 4 bündig gegen das Knochenlager 6 gepreßt wird.

In Phase (c) ist der Raum 5 zwischen Schaft 3 und Knochenlager 6 vollständig mit Knochenzement 11b hinterfüllt und die Hülle 4 vollflächig und bündig gegen das Knochenlager 6 gepreßt.

Das Ausmaß der Anpressung kann über den auf den Zement beaufschlagten Druck gesteuert werden. Durch diese Druckbeaufschlagung wird sowohl der Zement verdichtet und seine mechanischen Eigenschaften weiter verbessert, als auch der Verbund zwischen Zementmantel und Prothese, insbesondere bei rauhen oder oberflächenprofilierten Prothesen, verstärkt.

Von besonderer Bedeutung ist, daß während der gesamten Implantation der Kontaktbereich von Prothese und Zement vor Verunreinigungen geschützt wird, so daß der Verbund in optimaler Weise ausgeführt werden kann.

Mit der Aushärtung der Zementfüllung ist der Zementierungsvorgang abgeschlossen.

Die Erfindung kann durch weitere Ausgestaltungen vorteilhaft modifiziert werden.

So ist es beispielsweise zweckmäßig, die Befestigung der Hülle im proximalen Bereich des Prothesenschaftes mittels der dort angebrachten bzw. anzubringenden Abstandshalter 7 vorzunehmen. Diese Abstandshalter, die zur Zentrierung, Ausrichtung und Fixierung der Prothese im proximalen Bereich dienen, können separate Formkörper sein. Sie können aber etwa auch als Erhebungen, Wulste oder in Form eines umlaufenden Kragens in die Prothese selbst eingearbeitet sein. Entsprechende Ausgestaltungsformen sind aus dem einschlägigen Stand der Technik bekannt.

Weiterhin kann es zweckmäßig sein, die Hülle im distalen Bereich mit dem Markraumsperrer 8 zu verbinden. In diesem Fall können Markraumsperrer und Prothese in einem Arbeitsgang in den Femur eingesetzt werden.

Auch der Markraumsperrer selbst kann gegebenenfalls mit einer Zentriervorrichtung für die Prothese versehen bzw. so gestaltet sein, daß beim Einsetzen der Femurprothese dessen distales Ende durch den Markraumsperrer lagefixiert wird. Entsprechende Ausgestaltungsformen für Markraumsperrer und gegebenenfalls Prothesenschaftspitze sind aus dem einschlägigen Stand der Technik bekannt.

Die Herstellung derartiger Polymermaterialien und deren Verarbeitung zu Folien bzw. Folienprodukten ist bekannt. Für die vorliegende Erfindung werden diese Materialien zweckmäßigerweise zu Folienschläuchen bzw. -strümpfen verarbeitet, deren Formgebung so gestattet ist, daß sie dem jeweiligen Prothesenschaft angepaßt sind und auf diese eng anliegend aufgezogen werden können.

Die vorliegende Erfindung besitzt einen weiteren Vorzug dahingehend, daß es möglich ist, das Hüllenmaterial mit dem Heilungsvorgang förderlichen Zusätzen zu modifzieren. Derartige Zusätze können etwa osteoinduktive und/oder osteokonduktive Füllstoffe sein. Beispiele hierfür sind Kalziumverbindungen wie Calciumoxid, Calciumcarbonat und Calciumphosphate, insbesondere Hydroxylapatit und Tricalciumphosphat. Diese Materialien sind für das Knochenwachstum und insbesondere die Bildung von mineralisierter Knochenmatrix förderlich und begünstigen dadurch die Einheilung des Implantates. Weiterhin ist es vorteilhaft das Hüllenmaterial mit pharmazeutischen, wie insbesondere antibiotisch und/oder knochenwachstumsfördernden Wirkstoffen zu imprägnieren. Beispiele hierfür sind Antibiotika wie Gentamicin und Clindamycin sowie peptidische Wachstumsfaktoren wie insbesondere FGF und jene der BMP-Reihe.

Der besondere Vorteil liegt hierbei darin, daß die Wirkstoffe gezielt da plaziert werden können, wo sie benötigt werden und ihre Wirkung zweckentsprechend entfalten können, nämlich im Kontaktbereich zum Knochenbett. Im Vergleich zu üblichen Implantationstechniken mit wirkstoffbeladenen Knochenzementen kann hier durch die optimale Lokalisierung die Wirkstoffdosis verringert werden.

In einer besonders bevorzugten Ausführungsform kann die Erfindung in Form eines Implantationssets ausgestaltet sein. Ein derartiges Implantationsset besteht aus einer Femurprothese nach den Ansprüchen 1-5 in Packungseinheit mit einem gebrauchsfertigen bzw. zur Applikation vorbereiteten Knochenzement.

In einer weiteren Ausgestaltung kann bei diesem Implantationsset am distalen Ende der Hülle ein Markraumsperrer mit gegebenenfalls Zentriervorrichtung für die Prothese angebracht sein.

In diesen Ausgestaltungsformen werden dem Operateur alle erforderlichen erfindungsgemäßen Teile in praxisgerechter Form an die Hand gegeben, die er braucht, um die Implantation vornehmen zu können.

## Patentansprüche

1. Femurprothese (1) für die Implantation mittels Zementierung, wobei deren Schaft (2) eine von proximal bis distal reichende Längsbohrung (3) zur transprothetischen Applikation des Knochenzements aufweist und von einer dehnbaren Hülle (4) aus einem biokompatiblen Polymermaterial umgeben ist, dadurch gekennzeichnet, daß die Hülle, die aus einem bioresorbierbaren Polymermaterial besteht, Poren aufweist, die sich bei der Hinterfüllung der Hülle mit Knochenzement aufweiten, so daß der Zement mit dem Knochenlager in Kontakt tritt.

2. Femurprothese nach Anspruch 1, dadurch gekennzeichnet, daß die Hülle im proximalen Bereich an Abstandshaltern (7), die zur Zentrierung und Ausrichtung der Prothese dienen, befestigt ist.

3. Femurprothese nach den Ansprüchen 1 oder 2, dadurch gekennzeichnet, daß die Hülle im distalen Bereich an einem Markraumsperrer (8) befestigt ist.

4. Femurprothese nach den Ansprüchen 1 bis 3, dadurch gekennzeichnet, daß die Hülle aus einem Polymermaterial besteht, daß osteoinduktive und/oder osteokonduktive Füllstoffe enthält.

5. Femurprothese nach den Ansprüchen 1 bis 4, dadurch gekennzeichnet, daß die Hülle mit antibiotischen und/oder knochenwachstumsfördernden Wirkstoffen imprägniert ist.

6. Implantationsset bestehend aus einer Femurprothese nach den Ansprüchen 1 bis 5, dadurch gekennzeichnet, daß das Implantationsset in Packungseinheit einen gebrauchsfertigen bzw. zur Applikation vorbereiteten Knochenzement enthält.

7. Implantationsset nach Anspruch 6, dadurch gekennzeichnet, daß am distalen Ende der Hülle ein Markraumsperrer mit gegebenenfalls Zentriervorrichtung für die Prothese angebracht ist.

## Claims

1. Femoral prosthesis (1) for implantation by cementation, wherein the stem (2) thereof has a longitudinal borehole (3) extending proximally to distally for the transprosthetic application of the bone cement and is surrounded by an elastic sheath (4) of a biocompatible polymeric material characterized in that the sheath, which consists of a bioabsorbable polymeric material, has pores which widen on filling the sheath with bone cement so that the cement comes into contact with the bone bed.

2. Femoral prosthesis according to Claim 1, characterized in that the sheath is fastened in the proximal region to spacers (7) which are used to centre and align the prosthesis.

3. Femoral prosthesis according to Claims 1 or 2, characterized in that the sheath is fastened in the distal region to a medullary space blocker (8).

4. Femoral prosthesis according to Claims 1 to 3, characterized in that the sheath consists of a polymeric material which contains osteoinductive and/or osteoconductive fillers.

5. Femoral prosthesis according to Claims 1 to 4, characterized in that the sheath is impregnated with agents which are antibiotics and/or bone growth promoters.

6. Implantation kit consisting of a femoral prosthesis according to Claims 1 to 5, characterized in that the implantation kit contains in the pack unit a bone cement which is ready for use or is prepared for application.

7. Implantation kit according to Claim 6, characterized in that a medullary space blocker with, where appropriate, centring device for the prosthesis is attached at the distal end of the sheath.

## Revendications

1. Prothèse du fémur (1) pour l'implantation au moyen d'un cimentage, dans laquelle son fût (2) présente un perçage longitudinal (3), qui s'étend de l'arrière jusqu'à l'avant, pour l'application du ciment pour os à travers la prothèse et est entouré d'un manchon extensible (4) en matériau polymère biocompatible, caractérisée par le fait que le manchon, qui est constitué d'un matériau polymère biorésorbable, présente des pores qui s'élargissent lors du remplissage du manchon avec du ciment pour os de sorte que le ciment vient en contact avec l'appui osseux.

2. Prothèse du fémur selon la revendication 1, caractérisée par le fait que dans la zone arrière, le manchon est fixé à des écarteurs (7) qui servent à centrer et à orienter la prothèse.

3. Prothèse du fémur selon la revendication 1 ou 2, caractérisée par le fait que dans la zone avant, le manchon est fixé à un organe de blocage dans l'espace médullaire (8).

4. Prothèse du fémur selon les revendications 1 à 3, caractérisée par le fait que le manchon est constitué d'un matériau polymère qui contient des charges présentant une induction osseuse et/ou une conduction osseuse.

5. Prothèse du fémur selon les revendications 1 à 4, caractérisée par le fait que le manchon est imprégné de matériaux antibiotiques et/ou de matériau favorisant la croissance de l'os.

6. Set d'implantation constitué d'une prothèse du fémur selon la revendication 1 à 5, caractérisé par le fait que le set d'implantation contient dans un emballage unitaire un ciment pour os prêt à l'emploi ou préparé pour application.

7. Set d'implatation selon la revendication 6, caractérisé par le fait qu'à l'extrémité avant du manchon est rapporté un organe de blocage dans l'espace médullaire avec éventuellement un dispositif de centrage pour la prothèse.
